(19) Europäisches Patentamt
European Patent Office
Office européen des brevets

(11) Veröffentlichungsnummer: **0 110 830**
**B1**

(12) # EUROPÄISCHE PATENTSCHRIFT

(45) Veröffentlichungstag der Patentschrift: **20.11.86**

(21) Anmeldenummer: **83810539.3**

(22) Anmeldetag: **21.11.83**

(51) Int. Cl.⁴: **C 07 C 39/15**, C 08 L 25/06, C 08 L 55/02, C 10 M 129/10 // C09K15/08

(54) **Neue benzylierte Phenole.**

(30) Priorität: **25.11.82 CH 6875/82**

(43) Veröffentlichungstag der Anmeldung: **13.06.84 Patentblatt 84/24**

(45) Bekanntmachung des Hinweises auf die Patenterteilung: **20.11.86 Patentblatt 86/47**

(84) Benannte Vertragsstaaten: **BE DE FR GB IT NL**

(56) Entgegenhaltungen:
**EP - A - 0 081 457**
**CH - A - 633 505**
**GB - A - 2 041 357**

**GERALD SCOTT "Developments in polymer stabilisation-4", 1981, APPLIED SCIENCE PUBLISHERS LTD., London, Seiten 149-163**

(73) Patentinhaber: **CIBA-GEIGY AG, Klybeckstrasse 141, CH-4002 Basel (CH)**

(72) Erfinder: **Rosenberger, Siegfried, Im Gehracker 11, CH-4125 Riehen (CH)**

**EP 0 110 830 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft in ortho- und meta-Stellung, oder in para- und meta-Stellung dibenzylierte Phenole, deren Herstellung, deren Verwendung als Stabilisatoren für organisches Material sowie mit deren Hilfe stabilisiertes organisches Material.

In der Publikation von H. Budzikiewicz und J. Swoboda in Chem. Ber. 98 (10), 3264 (1965) sind benzylierte Phenole beschrieben, ohne auf deren Verwendbarkeit hinzuweisen. Benzylierte Phenole sind auch als Antioxidantien beschrieben worden, so z. B. in US-PS 3346648, DE-PS 2138839 oder JP-Anmeldung Nr. 69-40583.

Die bekannten Verbindungen genügen jedoch nicht in jeder Hinsicht den hohen Anforderungen, welche an einen hochwertigen Stabilisator gestellt werden. Die erfindungsgemässen ortho/meta- oder para/meta-benzylierten Phenole zeigen als Antioxidantien eine verbesserte Wirksamkeit, bei ausgezeichnetem Farbverhalten und Verträglichkeit mit den zu schützenden Substraten.

Gegenstand der Erfindung sind Verbindungen der allgemeinen Formel I

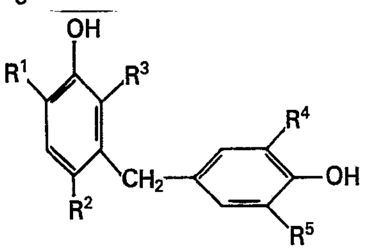 (I),

worin einer der Reste $R^1$ und $R^2$ eine Gruppe der Formel II

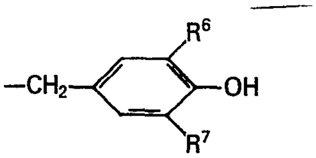 (II)

und der andere Methyl bedeutet, und $R^3$, $R^4$, $R^5$, $R^6$ und $R^7$ unabhängig voneinander $C_1$–$C_{12}$-Alkyl, $C_5$–$C_8$-Cycloalkyl, Phenyl, Naphthyl, $C_7$–$C_9$-Aralkyl oder $C_7$–$C_{19}$-Alkaryl sind.

Bedeuten $R^3$, $R^4$, $R^5$, $R^6$ und $R^7$ $C_1$–$C_{12}$-Alkyl, und bevorzugt $C_1$–$C_4$-Alkyl, so kann es sich z. B. um Methyl, Ethyl, n-Propyl, Isopropyl, n-Butyl, sec.-Butyl, tert.-Butyl, Isobutyl, n-Pentyl, tert-Amyl, n-Hexyl, n-Octyl, 1,1,3,3-Tetramethylbutyl, 2-Ethylhexyl, geradkettiges oder verzweigtes Nonyl, Decyl, Undecyl oder Dodecyl handeln. Bevorzugt sind $R^4$ und $R^6$ Methyl oder tert.-Butyl und $R^5$ und $R^7$ tert.-Butyl. Die bevorzugte Bedeutung von $R^3$ ist Methyl.

$R^3$, $R^4$, $R^5$, $R^6$ und $R^7$ sind als $C_5$–$C_8$-Cycloalkyl, z. B. Cyclopentyl, Cycloheptyl, Cyclooctyl und bevorzugt Cyclohexyl.

Bedeuten die Substituenten $R^3$, $R^4$, $R^5$, $R^6$ und $R^7$ $C_7$–$C_{10}$ Aralkyl, so handelt es sich z. B. um Benzyl, 1-Phenylethyl, $\alpha,\alpha$-Dimethylbenzyl oder um 2-Phenylethyl.

Als $C_7$–$C_{10}$ Alkaryl sind $R^3$, $R^4$, $R^5$, $R^6$ und $R^7$ beispielsweise o-Tolyl, m-Tolyl, p-Tolyl, 2,4-Dimethylphenyl, 2,6-Dimethylphenyl, 2,6-Diethylphenyl oder 4-tert.-Butylphenyl. Bevorzugt ist 2,4-Dimethylphenyl.

In bevorzugten Verbindungen haben die Substituenten $R^4$ und $R^6$ bzw. $R^5$ und $R^7$ die gleiche Bedeutung.

Bevorzugt werden Verbindungen der Formel I, worin einer der Reste $R^1$ und $R^2$ eine Gruppe der Formel III

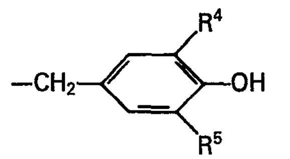 (III)

ist, und der andere Methyl bedeutet, und $R^3$, $R^4$ und $R^5$ unabhängig voneinander $C_1$–$C_4$-Alkyl, Cyclohexyl, Phenyl, $C_7$–$C_9$-Aralkyl oder $C_7$–$C_{10}$-Alkaryl sind.

Besonders bevorzugt sind Verbindungen der Formel I, worin einer der Reste $R^1$ und $R^2$ eine Gruppe der Formel III ist und der andere Methyl bedeutet, und $R^3$, $R^4$ und $R^5$ unabhängig voneinander $C_1$–$C_4$-Alkyl sind.

Von besonderem Interesse sind Verbindungen der Formel I, worin einer der Reste $R^1$ und $R^2$ eine Gruppe der Formel III ist und der andere Methyl bedeutet, $R^3$ $C_1$–$C_4$-Alkyl ist, $R^4$ Methyl oder t.-Butyl und $R^5$ tert.-Butyl bedeutet.

Beispiele von Verbindungen der Formel I sind:

2,6-Dimethyl-3,4-di-(3′,5′-di-tert.butyl-4′-hydroxybenzyl)-phenol
2,4-Dimethyl-3,6-di-(3′,5′-di-tert.butyl-4′-hydroxybenzyl)-phenol
2,6-Dimethyl-3,4-di-(3′-methyl-5′-tert.butyl-4′-hydroxybenzyl)-phenol
2,4-Dimethyl-3,6-di-(3′-methyl-5′-tert.butyl-4′-hydroxybenzyl)-phenol
2,6-Dimethyl-3-(3′,5′-di-tert.butyl-4′-hydroxybenzyl)-4-(3′-methyl-5′-tert.butyl-4′-hydroxybenzyl)phenol
2,6-Dimethyl-3-(3′-methyl-5′-tert.butyl-4′-hydroxybenzyl)-3-(3′-5′-di-tert.butyl-4′-hydroxybenzyl)-phenol
2,4-Dimethyl-3-(3′-5′-di-tert.butyl-4′-hydroxybenzyl)-6-(3′-methyl-5′-tert.butyl-4′-hydroxybenzyl)-phenol
2,4-Dimethyl-3-(3′-methyl-5′-tert.butyl-4′-hydroxybenzyl)-6-(3′-5′-di-tert.butyl-4′-hydroxybenzyl)-phenol
2-Ethyl-6-methyl-3,4-di-(3′,5′-di-tert.butyl-4′-hydroxybenzyl)-phenol
2-Butyl-4-methyl-3,6-di-(3′,5′-di-tert.butyl-4′-hydroxybenzyl)-phenol
2,6-Dimethyl-3,4-di-(3′,5′-dicyclohexyl-4′-hydroxybenzyl)-phenol
2,6-Dimethyl-3,4-di-(3′,5′)-dibenzyl-4′-hydroxybenzyl)-phenol

Die Verbindungen der Formel I werden nach an sich bekannten Benzylierungsreaktionen hergestellt. Dies geschieht z. B. durch Umsetzung ungefähr eines Mols eines Phenols der Formel IV oder V

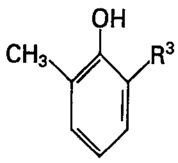 (IV) oder 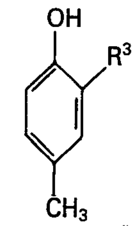 (V)

worin $R^3$ die oben angegebene Bedeutung hat mit ungefähr einem Mol einer Benzylierungskomponente der Formel VI

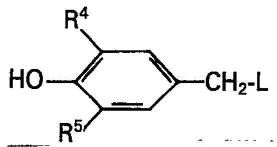 (VI)

und ungefähr einem Mol einer Benzylierungskomponente der Formel VII

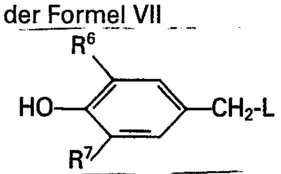 (VII)

worin L Hydroxy, $C_1$–$C_4$-Alkoxy oder Di-($C_1$–$C_4$)alkylamino bedeutet, und $R^4$, $R^5$, $R^6$ und $R^7$ die oben angegebene Bedeutung haben.

Die Reaktionen können gegebenenfalls in Gegenwart von Basen (z.B. tert.-Amine; Hydroxide, Amide, Carbonate von Alkali- oder Erdalkalimetallen), gegebenenfalls aber auch in Gegenwart saurer Katalysatoren (wie $H_2SO_4$, HCl, p-Toluolsulfonsäure, $BF_3$-Etherat, $ZnCl_2$, $CaCl_2$ oder $AlCl_3$ durchgeführt werden.

Das Verfahren kann mit oder ohne Lösungsmittel durchgeführt werden. Wird ein Lösungsmittel verwendet, so ist es ein inertes Lösungsmittel, wie Alkane (z.B. Hexan, Petrolether, Testbenzin), aromatische Kohlenwasserstoffe (z.B. Benzol, Toluol, Xylol), Ether (wie Diethylether, Diisopropylether, Dibutylether), Dimethylformamid, Dimethylacetamid oder Dioxan. Ferner sind als Lösungsmittel auch Alkohole (wie Methanol, Isopropanol, Butanol) sowie Chloralkane (wie Methylenchlorid) geeignet.

Die Reaktion kann bei Temperaturen zwischen 20 und 160 °C durchgeführt werden. Die obere Grenze wird bevorzugt durch die Rückflusstemperatur des gewählten Lösungsmittels gegeben.

Die Ausgangsstoffe der Formeln IV, V, VI und VII sind bekannte Substanzen, deren Herstellung dem Fachmann geläufig ist. Es befinden sich darunter verschiedene im Handel erhältliche Verbindungen.

Ein weiterer Gegenstand der Erfindung ist die Verwendung der Verbindungen der Formel I als Stabilisatoren für organisches Material gegen dessen Schädigung durch Einwirkung von Sauerstoff, Wärme, Licht und energiereiche Strahlung, wie z.B. β- und γ-Strahlung.

Vorzugsformen dieser erfindungsgemässen Verwendung sind die Verwendung der Verbindungen als Stabilisatoren in organischen Polymeren, insbesondere in Pfropfpolymeren auf der Basis Acrylnitril, Butadien, Styrol (ABS) oder in schlagfestem Polystyrol.

Weitere Beispiele für organisches Material, welches mit den erfindungsgemässen Verbindungen vorteilhaft stabilisiert werden kann, sind:

1. Polymere von Mono- und Diolefinen, beispielsweise Polyethylen (das gegebenenfalls vernetzt sein kann), Polypropylen, Polyisobutylen, Polybuten-1, Polymethylpenten-1, Polyisopren oder Polybutadien sowie Polymerisate von Cycloolefinen wie z.B. von Cyclopenten oder Norbornen.

2. Mischungen der unter 1) genannten Polymere, z.B. Mischungen von Polypropylen mit Polyethylen oder mit Polyisobutylen.

3. Copolymere von Mono- und Diolefinen untereinander oder mit anderen Vinylmonomeren, wie z.B. Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere) sowie Terpolymere von Ethylen mit Propylen und einem Dien, wie Hexadien, Dicyclopentadien oder Ethylidennorbornen.

4. Polystyrol.

5. Copolymere von Styrol oder α-Methylstyrol mit Dienen oder Acrylderivaten, wie z.B. Styrol-Butadien, Styrol-Acrylnitril-Methylacrylat; Mischungen von hoher Schlagzähigkeit aus Styrol-Copolymeren und einem anderen Polymer wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol, wie z.B. auf Polybutadien, Styrol und Acrylnitril auf Polybutadien, Styrol und Maleinsäureanhydrid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren sowie deren Mischungen mit den unter 5) genannten Copolymeren, wie z.B. als sogenannte ABS, MBS oder AES-Polymere bekannt sind.

7. Halogenhaltige Polymere, wie z.B. Polychloropren, Chlorkautschuk, chloriertes oder chlorsulfoniertes Polyethylen, Epichlorhydrin-homo- und copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere wie Vinylchlorid-Vinylidenchlorid, Vinylchlorid-Vinylacetat oder Vinylidenchlorid-Vinylacetat.

8. Polymere, die sich von α,β-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Compolymere, Acrylnitril-Alkoxyalkylacrylat-Copolymere, Acrylnitril-Vinylhalogenid-Copolymere od. Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat-, -stearat, -benzoat, -maleat, Polyvinylbutyral, Polyallylphthalat, Polyallylmelamin.

11. Homo- und Copolymere von cyclischen Ethern, wie Polyalkylenglykole, Polyethylenoxid, Polypropylenoxyd oder deren Copolymere mit Bisglycidylethern.

12. Polyacetale, wie Polyoxymethylen sowie solche Polyoxymethylene, die Comonomere wie z.B. Ethylenoxid enthalten.

12. Polyphenylenoxide und -sulfide sowie Mischungen von Polyphenylenoxiden mit Polystyrol.

14. Polyurethane, die sich von Polyethern, Polyestern und Polybutadienen mit endständigen Hydroxylgruppen einerseits und aliphatischen oder aromatischen Polyisocyanaten andererseits ableiten sowie deren Vorprodukte (Polyisocyanate, Polyole, Präpolymere).

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexamethylenterephthalamid, Poly-m-phenylen-isophthalamid sowie deren Copolymeren mit Poly-Ethern wie z.B. mit Polyethylenglykol, Polypropylenglykol oder Polytetramethylenglykol.

16. Polyharnstoffe, Polyimide und Polyamidimide.

17. Polyester, die sich von Dicarbonsäuren und Diolen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Poly-[2,2-bis(4-hydroxyphenyl)-propan]terephthalat, Polyhydroxybenzoate sowie Block-Polyetherester, die sich von Polyethylen mit Hydroxygruppen, Dialkoholen und Dicarbonsäuren ableiten.

18. Polycarbonate.

19. Polysulfone und Polyethersulfone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen sowie Vinylverbindungen als Vernetzungsmittel ableiten, wie auch deren halogenhaltige, schwer brennbare Modifikationen.

23. Vernetzbare Acrylharze, die sich von substituierten Acrylsäureestern ableiten, wie z.B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z.B. von Bis-glycidylethern oder cycloaliphatischen Diepoxiden.

26. Natürliche Polymere, wie Cellulose, Naturkautschuk, Gelatine sowie deren polymerhomolog chemisch abgewandelte Derivate, wie Celluloseacetate, -propionate und -butyrate bzw. die Celluloseether, wie Methylcellulose.

27. Natürliche und synthetische organische Stoffe, die reine monomere Verbindungen oder Mischungen von solchen darstellen, beispielsweise Mineralöle, tierische und pflanzliche Fette, Öle und Wachse, oder Öle, Wachse und Fette auf Basis synthetischer Ester (z.B. Phtalate, Adipate, Phosphate oder Trimellithate) sowie Abmischungen synthetischer Ester mit Mineralölen in beliebigen Gewichtsverhältnissen, wie sie z.B. als Weichmacher für Kunststoffe oder als Spinnpräparationen Anwendung finden, sowie deren wässrigen Emulsionen.

28. Wässrige Emulsionen natürlicher oder synthetischer Kautschuke wie z.B. Naturkautschuk-Latex oder Latices von carboxylierten Styrol-Butadien-Copolymeren.

Von besonderer Bedeutung ist die Stabilisierung von Styrolpolymeren und Elastomeren, insbesondere ABS. Die erfindungsgemäss stabilisierten Polymere weisen ein ausgezeichnetes Farbverhalten auf, und es besteht beste Verträglichkeit dieser Stabilisatoren mit den Polymeren.

Die erfindungsgemässen Verbindungen der Formel I sind auch besonders vorteilhaft als Stabilisatoren für Schmiermittel geeignet.

Die Stabilisatoren werden z.B. in einer Konzentration von 0,01 bis 5 Gew.-%, berechnet auf das zu stabilisierende Material, zugesetzt. Vorzugsweise werden 0,1 bis 2,0, besonders bevorzugt 0,2 bis 0,6 Gew.-% der Verbindungen, berechnet auf das zu stabilisierende Material, in dieses eingearbeitet.

Die Einarbeitung kann nach der Polymerisation erfolgen, beispielsweise durch Einmischen der Verbindungen und gegebenenfalls weiterer Additive in die Schmelze nach den in der Technik üblichen Methoden, vor oder während der Formgebung, oder auch durch Aufbringen der gelösten oder dispergierten Verbindungen auf das Polymere, gegebenenfalls unter nachträglichem Verdunsten des Lösungsmittels.

Die Erfindung betrifft daher auch das durch Zusatz einer Verbindung der Formel I stabilisierte organische Material, das gegebenenfalls noch andere bekannte und übliche Zusätze enthalten kann. Das so stabilsierte Material kann in verschiedenster Form angewendet werden, z.B. als Folien, Fasern, Bändchen, Profile oder als Bindemittel für Lacke, Klebemittel oder Kitte.

Als weitere Additive, mit denen zusammen die erfindungsgemäss verwendbaren Stabilisatoren eingesetzt werden können, sind beispielsweise zu nennen:

1. Antioxidantien

1.1. Alkylierte Monophenole
2,6-Di-tert.butyl-4-methylphenol
2-Tert.butyl-4,6-dimethylphenol
2,6-Di-tert.butyl-4-ethylphenol
2,6-Di-tert.butyl-4-n-butylphenol
2,6-Di-tert.butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.butyl-4-methoxymethylphenol
2,6-Diphenyl-4-octadecyloxyphenol

1.2. Alkylierte Hydrochinone
2,6-Di-tert.butyl-4-methoxyphenol
2,5-Di-tert.butyl-hydrochinon
2,5-Di-tert.amyl-hydrochinon

1.3. Hydroxylierte Thiodiphenylether
2,2′-Thio-bis-(6-tert.butyl-4-methylphenol)
2,2′-Thio-bis-(4-octylphenol)
4,4′-Thio-bis-(6-tert.butyl-3-methylphenol)
4,4′-thio-bis-(6-tert.butyl-2-methylphenol)

1.4. Alkyliden-Bisphenole
2,2′-Methylen-bis-(6-tert.butyl-4-methylphenol)
2,2′-Methylen-bis-(6-tert.butyl-4-ethylphenol)
2,2′-Methylen-bis-[4-methyl-6-(α-methylcyclohexyl)-phenol]
2,2′-Methylen-bis-(4-methyl-6-cyclohexylphenol)
2,2′-Methylen-bis-(6-nonyl-4-methylphenol)
2,2′-Methylen-bis-(4,6-di-tert.butylphenol)
2,2′-Ethyliden-bis-(4,6-di-tert.butylphenol)
2,2′-Ethyliden-bis-(6-tert.butyl-4-isobutylphenol)
4,4′-Methylen-bis-(2,6-di-tert.butylphenol)
4,4′-Methylen-bis-(6-tert.butyl-2-methylphenol)
1,1-Bis-(5-tert.butyl-4-hydroxy-2-methylphenyl)-butan
2,6-Di-(3-tert.butyl-5-methyl-2-hydoxybenzyl)-4-methylphenol
1,1,3-Tris-(5-tert.butyl-4-hydroxy-2-methylphenyl)-butan
1,1-Bis-(5-tert.butyl-4-hydroxy-2-methylphenyl)-3-n-dodecylmercaptobutan
Ethylenglycol-bis-[3,3-bis-(3′-tert.butyl-4′-hydroxyphenyl)-butyrat]
Di-(3-tert.butyl-4-hydroxy-5-methylphenyl)-dicyclopentadien
Di-[2-(3′-tert.butyl-2′-hydroxy-5′-methyl-benzyl)-6-tert.butyl-4-methyl-phenyl]-terephthalat

1.5. Benzylverbindungen
1,3,5-Tri-(3,5-di-tert.butyl-4-hydroxybenzyl)-2,4,6-trimethylbenzol
Di-(3,5-di-tert.butyl-4-hydroxybenzyl)-sulfid
3,5-di-tert.butyl-4-hydroxybenzyl-mercaptoessigsäure-isooctylester
Bis-(4-tert.butyl-3-hydroxy-2,6-dimethylbenzyl)dithiol-terephthalat
1,3,5-Tris-(3,5-di-tert.butyl-4-hydroxybenzyl)-isocyanurat
1,3,5-Tris-(4-tert.butyl-3-hydroxy-2,6-dimethylbenzyl)-isocyanurat
3,5-Di-tert.butyl-4-hydroxybenzyl-phosphonsäure-dioctadecylester
3,5-Di-tert.butyl-4-hydroxybenzyl-phosphonsäure-monoäthylester
Calcium-salz

1.6. Acylaminophenole
4-Hydroxy-laurinsäureanilid
4-Hydroxy-stearinsäureanilid
2,4-Bis-octylmercapto-6-(3,5-di-tert.butyl-4-hydroxyanilino)-s-triazin

1.7. Ester der β-(3,5-Di-tert.butyl-4-hydroxyphenyl)-propionsäure
mit ein- oder mehrwertigen Alkoholen, wie z.B. mit
Methanol,
Octadecanol,
1,6-Hexandiol
Neopentylglycol
Thiodiethylenglycol
Diethylenglycol
Triethylenglycol
Pentaerythrit
Tris-hydroxyethyl-isocyanurat
Di-hydroxyethyl-oxalsäurediamid

1.8. Ester der β-(5-Tert.butyl-4-hydroxy-3-methylphenyl)-propionsäure
mit ein- oder mehrwertigen Alkoholen, wie z.B. mit
Methanol
Octadecanol
1,6-Hexandiol
Neopentylglycol
Thiodiethylenglycol
Diethylenglycol
Triethylenglycol
Pentaerythrit
Tris-hydroxyethyl-isocyanurat
Di-hydroxyethyl-oxalsäurediamid

1.9. Amide der β-(3,5-Di-tert.butyl-4-hydroxyphenyl)-propionsäure
wie z.B.
N,N′-Di-(3,5-di-tert.butyl-4-hydroxyphenylpropionyl)-hexamethylendiamin
N,N′-Di-(3,5-di-tert.butyl-4-hydroxyphenylpropionyl)-trimethylendiamin
N,N′-Di-(3,5-di-tert.butyl-4-hydroxyphenylpropionyl)-hydrazin

2. UV-Absorber und Lichtschutzmittel

2.1. 2-(2′-Hydroxyphenyl)-benztriazole,
wie z.B. 5′-Methyl-, das 3′,5′-Di-tert.butyl-, 5′-Tert.butyl-, 5′-(1,1,3,3-Tetramethylbutyl)-, 5-Chlor-3′,5′-di-tert.butyl-, 5-Chlor-3′-tert.butyl-5′-methyl-, 3′-sec.Butyl-5′-tert.butyl-, 4′-Octoxy-, 3′,5′-Di-tert.amyl-.

2.2. 2-Hydroxybenzophenone,
wie z.B. das 4-Hydroxy-, 4-Methoxy-, 4-Octoxy-, 4-Decyloxy-, 4-Dodecyloxy-, 4-Benzyloxy-, 4,2′,4′-Trihydroxy-, 2′-Hydroxy-4,4′-dimethoxy-Derivat.

2.3. Ester von gegebenenfalls substituierten Benzoesäuren, wie z. B. 4-Tert.butyl-phenylsalicylat, Phenylsalicylyat, Octylphenylsalicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butyl-phenylester.

2.4. Acrylate, wie z. B. α-Cyan-β,β-diphenylacrylsäure-ethylester bzw. -isooctylester, α-Carbomethoxy-zimtsäure-methylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z. B. Nickelkomplexe des 2,2'-Thio-bis[4-(1,1,3,3-tetramethylbutyl)-phenols], wie der 1:1- oder der 1:2-Komplex, gegebenenfalls mit zusätzlichen Liganden wie n-Butylamin, Triethanolamin oder N-Cyclohexyl-diethanolamin, Nickeldibutyldithiocarbamat, Nickelsalze von 4-Hydroxy-3,5-di-tert.butylbenzyl-phosphonsäure-monoalkylestern wie vom Methyl- oder Ethylester, Nickelkomplexe von Ketoximen wie von 2-Hydroxy-4-methyl-phenyl-undecylketonoxim Nickelkomplexe des 1-Phenyl-4-lauroyl-5-hydroxy-pyrazols, gegebenenfalls mit zusätzlichen Lignanden.

2.6. Sterisch gehinderte Amine, wie z. B. Bis-(2,2,6,6-tetramethylpiperidyl)-sebacat, Bis-(1,2,2,6,6-pentamethylpiperidyl)-sebacat, n-Butyl-3,5-di-tert.butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-(2,2,6,6-Tetramethylpiperidyl)-hexamethylendiamin und 4-Tert.octylamino-2,6-dichlor-1,3,5-s-triazin, Tris-(2,2,6,6-tetramethylpiperidyl)-nitrilotriacetat.

2.7. Oxalsäurediamide, wie z. B. 4,4'-Di-octyloxy-oxanilid, 2,2'-Di-octyloxy-5,5'-di-tert.butyl-oxanilid, 2,2'-Di-dodecyloxy-5,5'-di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyl-oxanilid und dessen Gemisch mit 2-Ethoxy-2'-ethyl-5,4'-di-tert.butyl-oxanilid, Gemische von ortho- und para-Methoxy- sowie von o- und p-Ethoxy-di-substituierte Oxaniliden.

3. Metalldesaktivatoren, wie z. B. N,N'-Diphenyloxalsäurediamid, N-Salicylal-N'-salicyloylhydrazin, N,N'-Bis-salicyloylhydrazin, N,N'-Bis-(3,5-di-tert.butyl-4-hydroxyphenylpropionyl)-hydrazin, 3-Salicyloylamino-1,2,4-triazol, Bis-benzyliden-oxalsäuredihydrazid.

4. Phosphite und Phosphonite, wie z. B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythrit-diphosphit, Tris-(2,4-di-tert.butylphenyl)-phosphit, Diisodecyl-pentaerythrit-diphosphit, (Di-(2,4-di-tert.butylphenyl)-pentaerythrit-diphosphit, Tristearyl-sorbit-triphosphit, Tetrakis-(2,4-di-tert.butylphenyl)-4,4'-biphenylen-diphosphonit.

5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z. B. 4-tert.Butylbenzoesäure, Adipinsäure, Diphenylessigsäure.

9. Füllstoffe und Verstärkungsmittel, wie z. B. Calciumcarbonate, Silikate, Glasfasern, Asbest, Talk, Kaolin, Glimmer, Bariumsulfat, Metalloxide und -hydroxide, Russ, Graphit.

10. Sonstige Zusätze, wie z. B. Weichmacher, Gleitmittel, Emulgatoren, Pigmente, Optische Aufheller, Flammschutzmittel, Antistatika, Treibmittel.

Beispiel 1

2,6-Dimethyl-3,4-di-(3',5'-di-t-butyl-4'-hydroxybenzyl)-phenol

12,2 g 2,6-Dimethylphenol werden in 100 ml Methanol gelöst und mit 18 g 80%iger Schwefelsäure versetzt. Dann werden unter Rühren in Stickstoffatmosphäre innert 2 h bei 50 °C 50 g 4-Methoxymethyl-2,6-di-t-butylphenol in Portionen zugegeben. Das Reaktionsgemisch wird weitere 14 h bei 50 °C am Rückflusskühler gerührt. Anschliessend wird das ölige Reaktionsprodukt nach Ausfällen mit Wasser bei 20 °C in Toluol aufgenommen, die Toluollösung mit Wasser neutral gewaschen, mit $Na_2SO_4$ getrocknet und das Produkt des vorliegenden Beispiels aus der stark konzentrierten Toluollösung durch Chromatographieren an einer Kieselgel-Säule[1] rein abgetrennt. Das Produkt erstarrt kristallin. Smp. 155 °C.

[1] Fa. Merck, Darmstadt: «Kieselgel 60»; Korngrösse 70–230 mesh ASTM.

Beispiel 2

Ersetzt man in Beispiel 1 das 2,6-Dimethylphenol durch das isomere 2,4-Dimethylphenol, so erhält man bei sonst analoger Arbeitsweise das 2,4-Dimethyl-3,6-di-(3',5'-di-t-butyl-4'-hydroxybenzyl)-phenol in Form farbloser Kristalle vom Smp. 132 °C.

Beispiel 3

100 Gewichtsteile unstabilisiertes ABS-Pulver werden mit verschiedenen in den Tabellen I und II angegebenen Stabilisatoren gemischt.

Die erhaltenen Gemische werden auf einem Zweiwalzenstuhl während 5 min bei maximal 170 °C compoundiert und die Felle anschliessend abgezogen. Die Rohfelle werden auf einer hydraulischen Laborpresse bei 180 °C während 6 min zu 1 mm dicken Platten verpresst, aus welchen Prüflinge der Dimension 50×20 mm gestanzt werden.

Die Prüfung der Wirksamkeit der den Prüflingen zugesetzten Stabilisatoren wird durch Hitzealterung in einem Umluftofen bei 180 °C vorgenommen. Als Kriterium für die während der Alterung eingetretene Schädigung (Oxidation) dient das Infrarot-Absorptionsspektrum der Oberfläche, welches durch Reflexions-Spektroskopie (ATR) erhalten wird. Insbesondere wird die Zunahme der Carbonylextinktion (1720 $cm^{-1}$) als Funktion der Zeit verfolgt und mit einer konstant bleibenden Absorptionsbande (1455 $cm^{-1}$) verglichen. Dabei gilt als Mass für den Abbau:

$$V = \frac{\text{Opt. Dichte bei 1720 } cm^{-1} \ (> C = O}{\text{Opt. Dichte bei 1455 } cm^{-1} \ (> CH_2)}$$

Als willkürlicher Endpunkt wird die Zeit gewählt, nach welcher V den Wert 0,1 erreicht ($t_{0,1}$).

Tabelle I
Prüfung in ABS ohne Synergist.

| Stabilisator nach Beispiel Nr. | Ofenalterung 180 °C | | | | |
|---|---|---|---|---|---|
| | $t_{0,1}$ | Y.I. ASTM D 1925 nach Min. Ofenalterung | | | |
| 0,25 Gew.-% | | 0 | 30 | 60 | 90 |
| 1 | 54' | 21 | 31 | 39 | 63 |
| 2 | 65' | 18 | 28 | 35 | 50 |
| ohne Stabilisator | 7' | 17 | 53 | 73 | 83 |

Tabelle II
Prüfung in ABS mit dem Synergist DLTDP (Dilaurylthiodipropionat).

| Stab. nach Beispiel Nr. + DLTDP | Ofenalterung 180 °C | | | | | | |
|---|---|---|---|---|---|---|---|
| 0,25 Gew.-% Stab<br>0,5 Gew.-% DLTDP | $t_{0,1}$ | Y.I. ASTM nach Min. Ofenalterung | | | | | |
| | | 0 | 30 | 60 | 90 | 120 | 150 |
| 1 | 120' | 19 | 29 | 33 | 37 | 41 | 54 |
| 2 | 132' | 18 | 27 | 31 | 35 | 41 | 61 |
| ohne Stab. | 7' | 17 | 53 | 73 | 83 | | |

Beispiel 4

Ein schlagfestes Polystyrol mit 8 Gew.-% Polybutadien-Gehalt (high-cis) und mit 0,035 Gew.-% 2,6-Di-tert.-butyl-p-cresol als Grundstabilisator, 0,05 Gew.-% Zinkstearat als Gleitmittel sowie 0,1 Gew.-% eines der erfindungsgemässen Antioxidantien (in den nachfolgenden Tabellen III und IV jeweils mit der Nummer des entsprechenden Herstellungsbeispiels gekennzeichnet), wird zweimal bei 220 °C extrudiert und das erhaltene Granulat bei 185 °C in 3 min zu 2 mm dicken Prüfplättchen verpresst.

Die Prüflinge werden einer Ofenalterung im Umluftofen unterzogen und

a) Der Yellowness-Index gemäss ASTM D 1925 bei 80 °C (Messung der Prüflinge nach 0, 250, 500, 750 und 1000 h) und bei 160 °C (Messung der Prüflinge nach 0, 60, 90, 120 und 180 min) bestimmt. Die Resultate sind in Tabelle III wiedergegeben.

b) Die Schlagzähigkeit (SZ) in Kp. $cm/cm^2$ nach Alterung bei 160 °C (Messung der Prüflinge nach 30, 60, 120, 150, 180, 240, 300, 360, 420, 480 min) bestimmt. Letztere Resultate sind in Tabelle IV zusammengestellt.

Tabelle III

| Stab. nach Beispiel Nr. | Y.I. bei 80°C nach Stunden | | | | | Y.I. bei 160 °C nach Minuten | | | | |
|---|---|---|---|---|---|---|---|---|---|---|
| | 0 | 250 | 500 | 700 | 1000 | 0 | 60 | 90 | 120 | 180 |
| Ohne Stabilisator | 1 | 13 | 17 | 18 | 28 | 1 | 22 | 51 | 69 | 86 |
| 1 | 12 | 21 | 27 | 28 | 32 | 12 | 22 | 28 | 36 | 42 |

Tabelle IV

| Stab nach Beispiel Nr. | SZ nach Alterung bei 160 °C nach Minuten | | | | | | | |
|---|---|---|---|---|---|---|---|---|
| | 30 | 60 | 120 | 150 | 180 | 240 | 300 | 360 |
| Ohne Stabilisator | 10,4 | | | | | | | |
| 1 | X | X | X | X | X | X | X | 10,5 |

X bedeutet: Prüfling nicht gebrochen

**Patentansprüche**

1. Verbindungen der allgemeinen Formel I

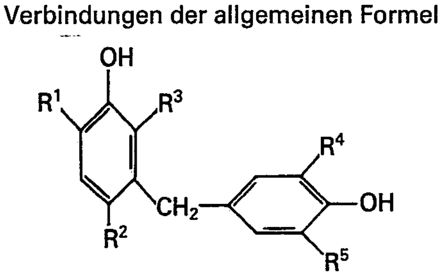

(I),

worin einer der Reste $R^1$ und $R^2$ eine Gruppe der Formel II

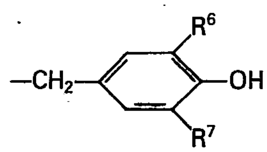

(II)

ist, und der andere Methyl bedeutet, und $R^3$, $R^4$, $R^5$, $R^6$ und $R^7$ unabhängig voneinander $C_1$–$C_{12}$-Alkyl, $C_5$–$C_8$-Cycloalkyl, Phenyl, Naphthyl, $C_7$–$C_9$-Aralkyl oder $C_7$–$C_{10}$-Alkaryl sind.

2. Verbindungen gemäss Anspruch 1 der Formel I, worin einer der Reste $R^1$ und $R^2$ eine Gruppe der Formel III

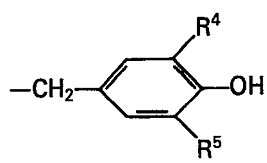

(III)

ist, und der andere Methyl bedeutet, und $R^3$, $R^4$ und $R^5$ $C_1$–$C_4$-Alkyl, Cyclohexyl, Phenyl, $C_7$–$C_9$-Aralkyl oder $C_7$–$C_{10}$-Alkaryl sind.

3. Verbindungen gemäss Anspruch 2 der Formel I, wobei $R^3$, $R^4$ und $R^5$ unabhängig voneinander $C_1$–$C_4$-Alkyl sind.

4. Verbindungen gemäss Anspruch 3 der Formel I, wobei $R^3$ $C_1$–$C_4$-Alkyl ist, $R^4$ Methyl oder tert.-Butyl und $R^5$ tert.-Butyl bedeutet.

5. Verbindungen gemäss Anspruch 1 der Formel I, worin $R^1$ Methyl ist.

6. Verbindungen gemäss Anspruch 1 der Formel I, worin $R^2$ Methyl ist.

7. Die Verbindung 2,6-Dimethyl-3,4-di-(3′,5′-di-tert.butyl-4′-hydroxybenzyl)-phenol gemäss Anspruch 1.

8. Die Verbindung 2,4-Dimethyl-3,6-di-(3′,5′-di-tert.butyl-4′-hydroxybenzyl)-phenol gemäss Anspruch 1.

9. Herstellung der Verbindungen der Formel I gemäss Anspruch 1, dadurch gekennzeichnet, dass man ungefähr ein Mol eines Phenols der Formel IV oder V

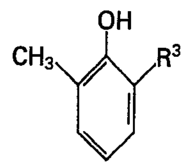

(IV) oder

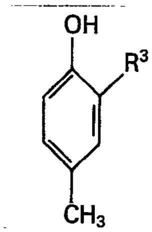

(V)

mit ungefähr einem Mol einer Benzylierungskomponente der Formel VI

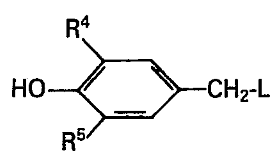

(VI)

und ungefähr einem Mol einer Benzylierungskomponente der Formel VII

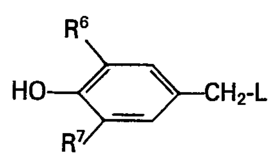

(VII)

worin L Hydroxy, $C_1$–$C_4$-Alkoxy oder Di-($C_1$–$C_4$)-alkylamino bedeutet und die übrigen Symbole wie im Anspruch 1 definiert sind, umsetzt.

10. Verwendung der Verbindungen gemäss Anspruch 1 der Formel I als Stabilisatoren für organisches Material.

11. Mit Verbindungen der Formel I gemäss Anspruch 1 stabilisiertes organisches Material.

12. Stabilisiertes organisches Polymer gemäss Anspruch 11.

13. Stabilisiertes Polystyrol oder Acrylnitril/Butadien/Styrol-Pfropfpolymer (ABS) gemäss Anspruch 12.

14. Stabilisiertes Schmiermittel gemäss Anspruch 11.

**Claims**

1. A compound of the general formula i

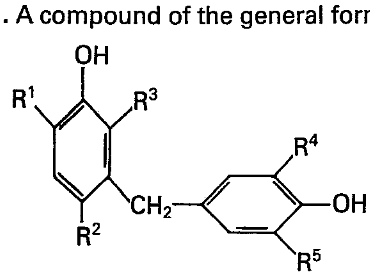

(I),

in which one of the radicals $R^1$ and $R^2$ is a group of the formula II

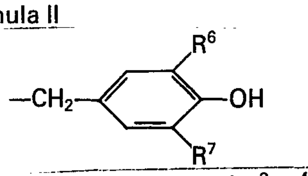 (II)

and the other is methyl, and $R^3$, $R^4$, $R^5$, $R^6$ and $R^7$ are each independently $C_1$–$C_{12}$-alkyl, $C_5$–$C_8$-cycloalkyl, phenyl, naphthyl, $C_7$–$C_9$-aralkyl or $C_7$–$C_{10}$-alkaryl.

2. A compound according to claim 1, of the formula I, in which one of the radicals $R^1$ and $R^2$ is a group of the formula III

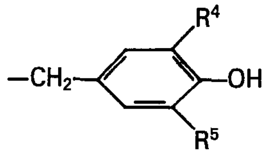 (III)

and the other is methyl, and $R^3$, $R^4$ and $R^5$ are $C_1$–$C_4$-alkyl, cyclohexyl, phenyl, $C_7$–$C_9$-aralkyl or $C_7$–$C_{10}$-alkaryl.

3. A compound according to claim 2, of the formula I, in which $R^3$, $R^4$ and $R^5$ are each independently $C_1$–$C_4$-alkyl.

4. A compound according to claim 3, of the formula I, in which $R^3$ is $C_1$–$C_4$-alkyl, $R_4$ is methyl or tert-butyl and $R^5$ is tert-butyl.

5. A compound according to claim 1, of the formula I, in which $R^1$ is methyl.

6. A compound according to claim 1, of the formula I, in which $R_2$ is methyl.

7. The compound 2,6-dimethyl-3,4-di-(3',5'-di-tert-butyl-4'-hydroxybenzyl)phenol according to claim 1.

8. The compound 2,4-dimethyl-3,6-di-(3',5'-di-tert-butyl-4'-hydroxybenzyl)phenol according to claim 1.

9. A process for the preparation of a compound of the formula I according to claim 1, which process comprises reacting about 1 mole of a phenol of the formula IV or V

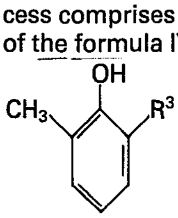 (IV) oder 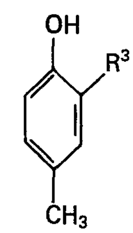 (V)

with about 1 mole of a benzylation component of the formula VI

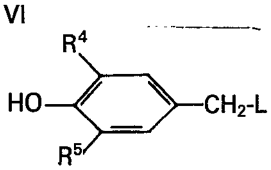 (VI)

and about 1 mole of a benzylation component of the formula VII

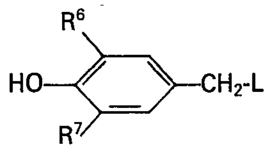 (VII)

in which formulae L is hydroxy, $C_1$–$C_4$-alkoxy or di($C_1$–$C_4$)alkylamino and the remaining symbols are as defined in claim 1.

10. Use of a compound according to claim 1, of the formula I, as stabiliser for organic material.

11. Organic material which has been stabilised with a compound of the formula I according to claim 1.

12. A stabilised organic polymer according to claim 11.

13. Stabilised polystyrene or acrylonitrile/butadiene/styrene graft polymer (ABS) according to claim 12.

14. A stabilised lubricant according to claim 11.

**Revendications**

1. Composés répondant à la formule générale I

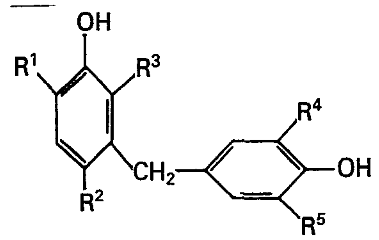 (I),

dans laquelle l'un des symboles $R^1$ et $R^2$ représente un radical de formule II

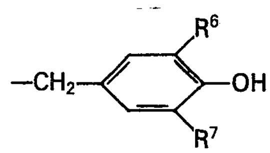 (II)

l'autre représente un radical méthyle, et $R^3$, $R^4$, $R^5$, $R^6$ et $R^7$ représentent chacun, indépendamment les uns des autres, un alkyle en $C_1$–$C_{12}$, un cycloalkyle en $C_5$–$C_8$, un phényle, un napthyle, un aralkyle en $C_7$–$C_9$ ou un alkylaryle en $C_7$–$C_{10}$.

2. Composés de formule I selon la revendication 1, dans lesquels l'un des symboles $R_1$ et $R_2$ représente un radical de formule III

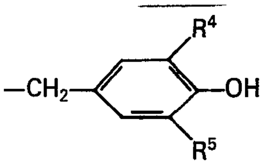 (III)

l'autre représente un radical méthyle, et $R^3$, $R^4$ et $R^5$ représentent chacun un alkyle en $C_1$–$C_4$, un cyclohexyle, un phényle, un aralkyle en $C_7$–$C_9$ ou un alkylaryle en $C_7$–$C_{10}$.

3. Composés de formule I selon la revendication 2, dans lesquels $R^3$, $R^4$ et $R^5$ représentent chacun, indépendamment les uns des autres, un radical alkyle contenant de 1 à 4 atomes de carbone.

4. Composés de formule I selon la revendication 3, dans lesquels $R^3$ représente un alkyle en $C_1$–$C_4$, $R^4$ un méthyle ou un tert-butyle et $R^5$ un tert-butyle.

5. Composés de formule I selon la revendication 1, dans lesquels $R^1$ représente un méthyle.

6. Composés de formule I selon la revendication 1, dans lesquels $R^2$ représente un méthyle.

7. Composé selon la revendication 1, en l'espèce le diméthyl-2,6-bis-(ditert-butyl-3,5-hydroxy-4-benzyl)-3,4-phénol.

8. Composé selon la revendication 1, en l'espèce le diméthyl-2,4-bis-(ditert-butyl-3,5-hydroxy-4-benzyl)-3,6-phénol.

9. Procédé de préparation des composés de formule I selon la revendication 1, procédé caractérisé en ce qu'on fait réagir environ 1 mol d'un phénol de formule IV ou de formule V

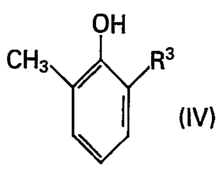

(IV) oder

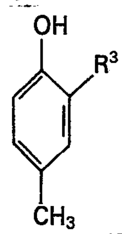

(V)

avec environ 1 mol d'une composante de benzylation de formule VI

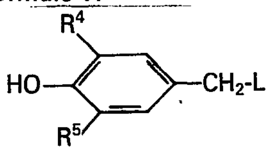

(VI)

et environ 1 mol d'une composante de benzylation de formule VII

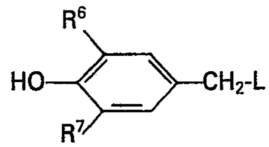

(VII)

formules dans lesquelles L représente un hydroxy, un alcoxy en $C_1$–$C_4$ ou un di-($C_1$–$C_4$)-alkylamino et les autres symboles ont les significations qui leur ont été données à la revendication 1.

10. Application des composés de formule I selon la revendication 1 comme stabilisants pour des matières organiques.

11. Matières organiques qui ont été stabilisées avec des composés de formule I selon la revendication 1.

12. Polymères organiques stabilisés selon la revendication 11.

13. Polystyrène stabilisé ou polymère greffé acrylonitrile/butadiène/styrène (ABS) stabilisé selon la revendication 12.

14. Lubrifiant stabilisé selon la revendication 11.